# EUROPEAN PATENT SPECIFICATION

(11) **EP 3 979 932 B1**
(45) Date of publication and mention of the grant of the patent: **17.04.2024**
(21) Application number: 20746694.7
(22) Date of filing: 17.06.2020
(51) Int. Cl.: A61B 17/70, A61F 2/44

(54) **BI-DIRECTIONAL MOTION SPINAL IMPLANT**
WIRBELSÄULENIMPLANTAT MIT BIDIREKTIONALER BEWEGUNG
IMPLANT RACHIDIEN À MOUVEMENT BIDIRECTIONNEL

(30) Priority: 23.06.2019 US 201916449403; 27.11.2019 US 201962940908 P
(43) Date of publication of application: 13.04.2022
(73) Proprietor: Premia Spine Ltd., 42504 Ramat Poleg (IL)
(72) Inventor: HAZIZA, Rafi, 2724024 Kiryat Bialik (IL); ARNIN, Uri, 3603117 Kiryat Tivon (IL)
(74) Representative: Wright, Howard Hugh Burnby
(86) International application number: PCT/IB2020/055657
(87) International publication number: WO 2020/261046

(56) References cited:
- EP-A1- 3 391 859
- WO-A1-2007/043044
- WO-A2-2006/020530
- WO-A2-2006/102443
- WO-A2-2008/134703
- US-A1- 2005 131 406
- US-A1- 2006 095 132
- US-A1- 2012 083 845
- US-A1- 2014 088 649

## Description

### FIELD OF THE INVENTION

The present invention relates generally to methods (not claimed) and apparatus for minimally invasive surgery on spinal structures, and particularly to a spinal implant that permits bi-directional motion for dynamic stabilization of adjacent vertebrae.

### BACKGROUND OF THE INVENTION

Posterior lumbar or transforaminal lumbar surgical procedures involve placement of a spinal implant secured by pedicle screws and neural decompression, all of which are done through posterior incisions, which are kept to a minimum in minimally invasive surgery.

For example, dynamic stabilization techniques have been developed for the posterior spine. These posterior techniques utilize pedicle screws and a dynamic rod. Typically the dynamic rod has a mechanism to bend under certain loads or forces, thereby absorbing some stress and strain that is applied to the spine.

US-A1-2005/131406 describes prostheses, systems, and methods for replacement of natural facet joints between adjacent vertebrae using polyaxial attachment mechanisms for securing the prostheses to the vertebrae.

WO-A1-2007/043044 describes a spinal prosthesis including a first spinal attachment member (12) attachable to a first posterior portion of a spinal structure, a second spinal attachment member (16) attachable to a second posterior portion of the spinal structure, the first and second posterior portions being adjacent superiorly-inferiorly to one another, and a connector element (20) attached to the first spinal attachment member.

WO-A2-2006/102443 describes methods and devices for repairing, replacing and/or augmenting natural facet joint surfaces and/or facet capsules. A facet joint restoration device of the invention for use in a restoring a facet joint surface comprises: a cephalad facet joint element comprising a flexible member adapted to engage a first vertebrae and an artificial cephalad j oint; and a caudad facet j oint element comprising a connector adapted for fixation to a second vertebrae and an artificial caudad joint adapted to engage the cephalad facet joint.

US-A1-2014/088649 describes a adjustable articulating spinal rod system including a first elongated element secured to a first bone, a second elongated element secured to the spine, and an articulating joint connecting the first and second elongated elements.

US-A1-2012/083845 describes a compound spinal rod and method for dynamic stabilization of the spine, the compound spinal rod including a first rod connected by a linkage to a second rod, the linkage allowing for movement of the first rod relative to the second rod.

US-A1-2006/095132 describes an implant having a first member having a first elongated channel and a second member having a second elongated concave channel that becomes generally opposed when the first and second members are mounted to a first and second vertebra. A ball bearing is received in each of the channels to both separate and maintain a predetermined distance between the first and second member and also to facilitate movement in a plane that intersects an axis of the spinal column.

### SUMMARY OF THE INVENTION

The present invention seeks to provide a spinal implant that permits bi-directional motion for dynamic stabilization of adj acent vertebrae, as described in more detail further below.

The present invention relates to a spinal implant as claimed hereafter. Preferred embodiments of the invention are set forth in the dependent claims. There is thus provided in accordance with a non-limiting embodiment of the present invention a spinal implant including first and second pedicle screws, each of which includes a threaded shank coupled to a head, and first and second cantilevered arms coupled to the first and second pedicle screws, respectively, wherein the first cantilevered arm includes a contact member arranged to contact and move over a contact portion of the second cantilevered arm, wherein said contact member comprises a round roller element which rolls about pivots in said first cantilevered arm.

In one embodiment, an outer contour of the head is convex, and each of the first and second cantilevered arms includes a concave inner portion, and the first and second cantilevered arms are secured to the first and second pedicle screws, respectively, with a fastener that presses the concave inner portion against the outer contour of the head.

In one embodiment, a fastener-interface portion of each of the first and second cantilevered arms is convex, and the fastener includes a concave inner portion configured to press against the fastener-interface portion.

In one embodiment, the first and second cantilevered arms are parallel to each other.

In one embodiment, the contact member is convex and the contact portion is concave.

In one embodiment, the second cantilevered arm includes at least one side wall that straddles the contact member.

In one embodiment, the contact member has a different hardness than the contact portion.

### BRIEF DESCRIPTION OF THE DRAWINGS

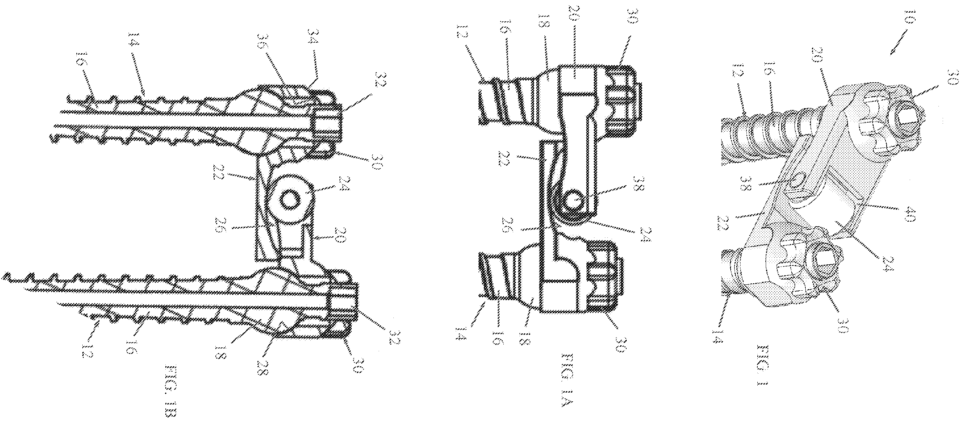

The embodiments of the invention are illustrated in fig. 1 to 3B.

The examples shown in the other figures do not form part of the invention but represent background art that is useful for understanding the invention.

The present invention will be understood and appreciated more fully from the following detailed description, taken in conjunction with the drawings in which:
Figs. 1, 1A and IB are simplified perspective, front-view and sectional illustrations of a spinal implant, constructed and operative in accordance with a non-limiting embodiment of the present invention;
Figs. 2, 2A and 2B are simplified perspective, front-view and sectional illustrations of a spinal implant, in accordance with another non-limiting embodiment of the present invention;
Figs. 3, 3A and 3B are simplified perspective, front-view and sectional illustrations of a spinal implant, in accordance with another non-limiting embodiment of the present invention;
Figs. 4, 4A and 4B are simplified perspective, front-view and sectional illustrations of a spinal implant (not claimed);
Figs. 5, 5A and 5B are simplified perspective, front-view and sectional illustrations of a spinal implant (not claimed);
Figs. 6, 6A and 6B are simplified perspective, front-view and sectional illustrations of a spinal implant (not claimed);
Figs. 7, 7A and 7B are simplified perspective, front-view and sectional illustrations of a spinal implant (not claimed);
Figs. 8 and 8A are simplified front-view and sectional illustrations of a spinal implant (not claimed); and
Figs. 9, 9A and 9B are simplified perspective, front-view and sectional illustrations of a spinal implant (not claimed).

### DETAILED DESCRIPTION OF EMBODIMENTS

Reference is now made to Figs. 1-1B, which illustrate a spinal implant 10, constructed and operative in accordance with a non-limiting embodiment of the present invention.

The spinal implant 10 includes first and second pedicle screws 12 and 14. Each screw includes a threaded shank 16 coupled to a head 18, which may be a polyaxial head. First and second cantilevered arms 20 and 22 are coupled to the first and second pedicle screws 12 and 14, respectively. The first cantilevered arm 20 includes a contact member 24 arranged to contact and move over a contact portion 26 of the second cantilevered arm 22.

The outer contour of head 18 may be convex. Each of the first and second cantilevered arms 20 and 22 includes a concave inner portion 28 (Fig. 1B) complementarily shaped to match the curvature of the head 18. The first and second cantilevered arms 20 and 22 may be secured to the first and second pedicle screws 12 and 14, respectively, with a fastener 30 that presses the concave inner portion 28 against the outer contour of the head 18. For example, in the illustrated embodiment, fastener 30 includes a male-threaded portion 32 (Fig. 1B) that engages female thread formed in an upper portion of head 18.

In one embodiment, a fastener-interface portion 34 (Fig. 1B) of each of the first and second cantilevered arms 20 and 22 is convex. The fastener 30 includes a concave inner portion 36 configured to press against the fastener-interface portion 34, as seen in Fig. 1B.

Due to the convex-concave interface between the fastener and the cantilevered arms and between the cantilevered arms and the pedicle screw head, the first and second cantilevered arms 20 and 22 may be secured to the first and second pedicle screws 12 and 14, respectively, at any angular orientation along the contour of the pedicle screw head 18. This provides the surgeon with limitless possibilities of mounting the spinal implant 10 in the patient first and second cantilevered arms 20 and 22 directed at any desired direction. For example, in the illustrations, the first and second cantilevered arms 20 and 22 are parallel to each other; however, they can be non-parallel, such as by tilting one or both of the arms over the outer contour of the head 18.

In the illustrated embodiment, the contact member 24 is convex and the contact portion 26 is concave. According to the invention, the contact member 24 is a round roller element, such as a cylindrical roller bearing element which rolls about pivots 38 and which may fit in a recess 40 formed in first cantilevered arm 20. Thus, in this embodiment, the contact member 24 is pivotally coupled to the first cantilevered arm 20.

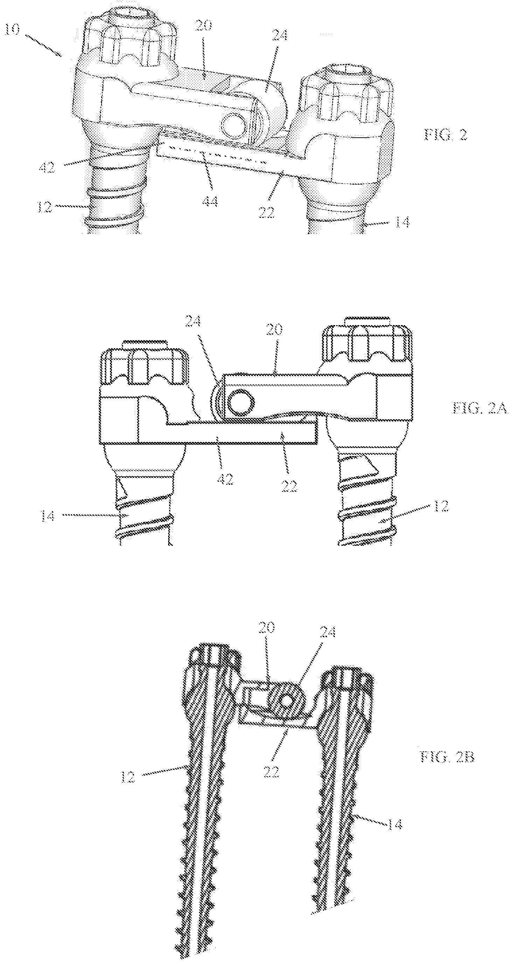

Reference is now made to Figs. 2-2B, which illustrate a modified version of the spinal implant 10, with like elements designated by like numerals. In this version, second cantilevered arm 22 includes at least one side wall 42 that straddles contact member 24. The first cantilevered arm 20 is arranged to move over second cantilevered arm 22 in two linear directions (back and forth) along a longitudinal axis 44 of the arms 20 and 22. In reality, other motion may also occur (e.g., perpendicular or otherwise tilted with respect to longitudinal axis 44), due to imperfections or manufacturing tolerances, or due to the chosen rotational orientation of the arms with respect to the round outer contours of heads 18. The side wall 42 may be useful to limit the non-longitudinal movement and ensure that contact member 24 of first cantilevered arm 20 does not slip off second cantilevered arm 22.

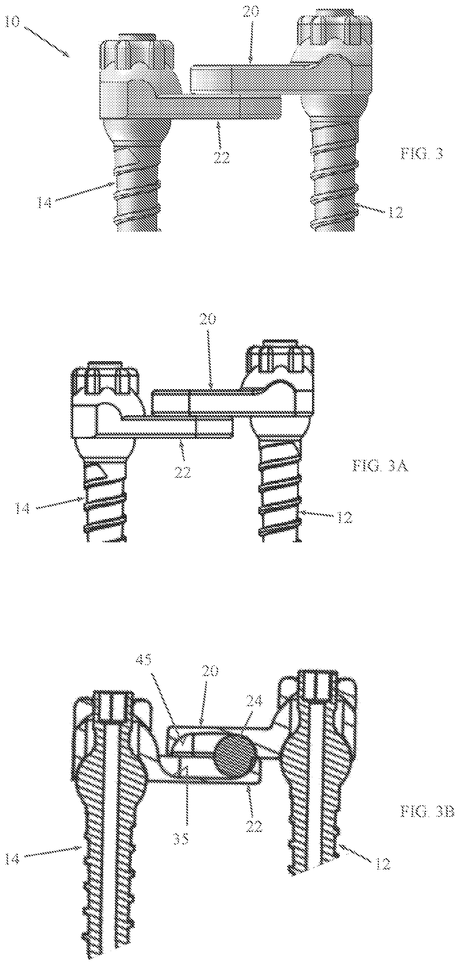

Reference is now made to Figs. 3-3B, which illustrate a modified version of the spinal implant 10, with like elements designated by like numerals. In this version, the contact member 24 (Fig. 3B), e.g., a sphere, is movable independently of both the first and second cantilevered arms 20 and 22. This is in contrast with the other embodiments, in which the contact member is fixedly coupled to the first cantilevered arm 20. In this embodiment, both the first and second cantilevered arms 20 and 22 may be formed with a concave inner portion 35 (Fig. 3B) to accommodate the shape of contact member 24.

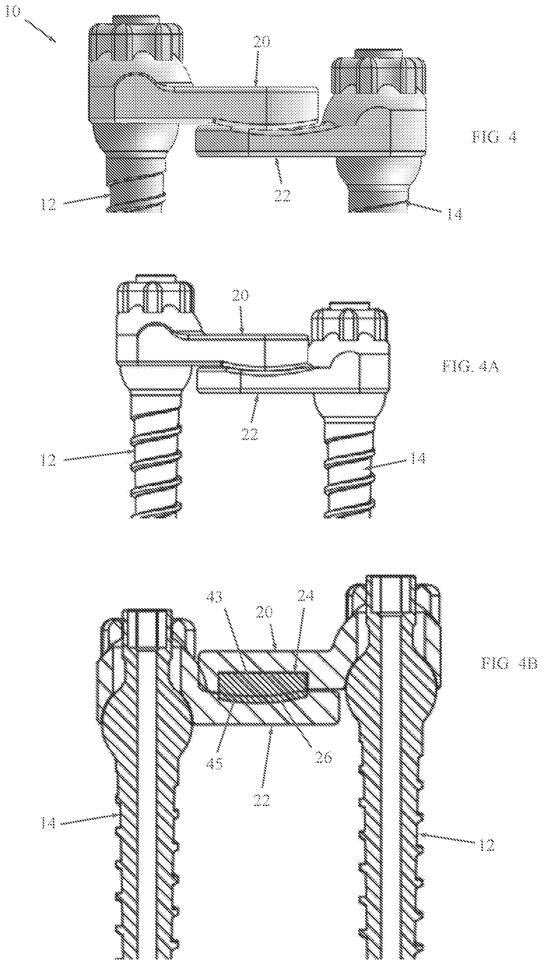

Reference is now made to Figs. 4-4B, which illustrate a modified version (not claimed) of the spinal implant 10, with like elements designated by like numerals. In this version, the contact member 24 (Fig. 4B) has an upper flat face 43 and a lower convex contact face 45 for contacting the concave contact portion 26 of the second cantilevered arm 22.

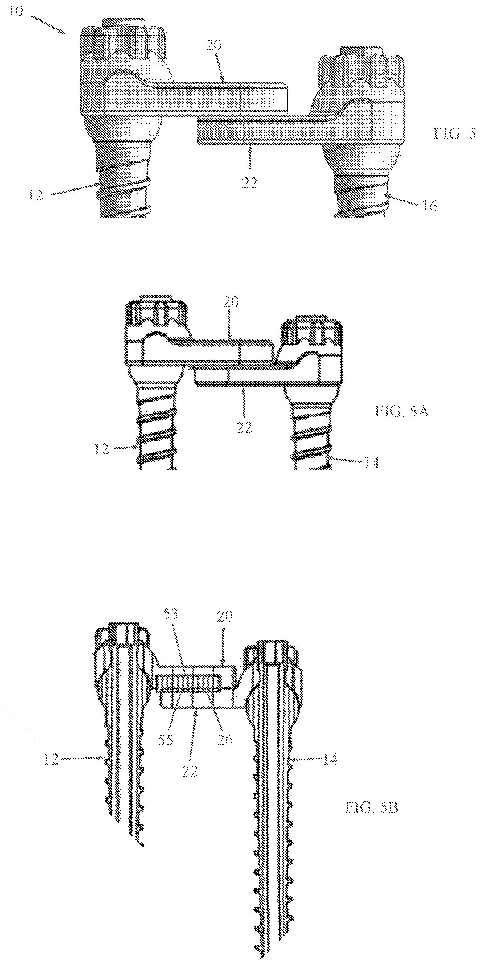

Reference is now made to Figs. 5-5B, which illustrate a modified version of the spinal implant 10, with like elements designated by like numerals. In this version, the contact member 24 (Fig. 5B) has an upper flat face 53 and a lower flat contact face 55 for contacting the flat contact portion 26 of the second cantilevered arm 22.

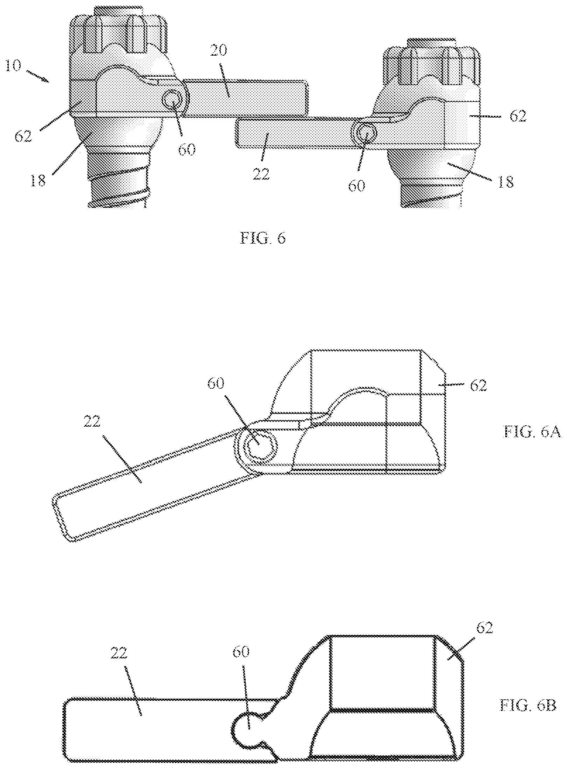

Reference is now made to Figs. 6-6B, which illustrate a modified version (not claimed) of the spinal implant 10, with like elements designated by like numerals. In this version, the first and second cantilevered arms 20 and 22 are each pivoted about a pivot 60 with respect to a base member 62 that fits over the head 18. The pivot 60 may be a ball-and-socket joint (as in Fig. 6B); additionally or alternatively it may be a locking screw (as in Fig. 6A) or a ratchet which can lock the first and second cantilevered arms 20 and 22 at any desired angle. The contact member 24 and the contact portion 26 are shown as flat but may be any of the other configurations of the other embodiments.

In all embodiments, the contact member 24 may have the same or a different hardness (softer or harder) than the contact portion 26.

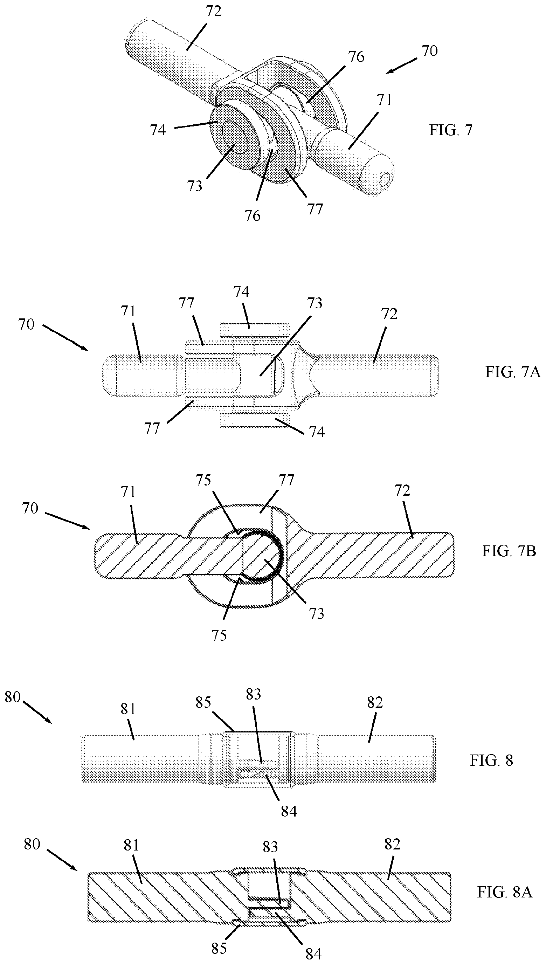

Reference is now made to Figs. 7, 7A and 7B, which illustrate a spinal implant 70 (not claimed).

As in the other examples, spinal implant 70 includes first and second cantilevered arms 71 and 72 that can be coupled to pedicle screws (the arms are structured as spinal rods to be received in the pedicle screw tulip head, for example). First cantilevered arm 71 has a transverse rod 73 with enlarged end faces 74. Transverse rod 73 (it is transverse to the shaft of arm 71 and it is the contact member) is arranged to contact and move over a contact portion 75 of second cantilevered arm 72. The contact portion 75 is the inner perimeters of a pair of oval or elliptical apertures 76 formed in a yoke member 77 extending from arm 72.

Reference is now made to Figs. 8 and 8A, which illustrate a spinal implant 80 (not claimed). Here again, spinal implant 80 includes first and second cantilevered arms 81 and 82 that can be coupled to pedicle screws (the arms are structured as spinal rods to be received in the pedicle screw tulip head, for example). First cantilevered arm 81 has a contact member 83 that axially protrudes out of an end of arm 81. Contact member 83 may be a flat or convex plate that contacts and slides over a contact portion 84 that axially protrudes out of an end of arm 82. The contact portion 84 may be flat or concave, for example. The contact member 83 and the contact portion 84 may be surrounded by a ring 85.

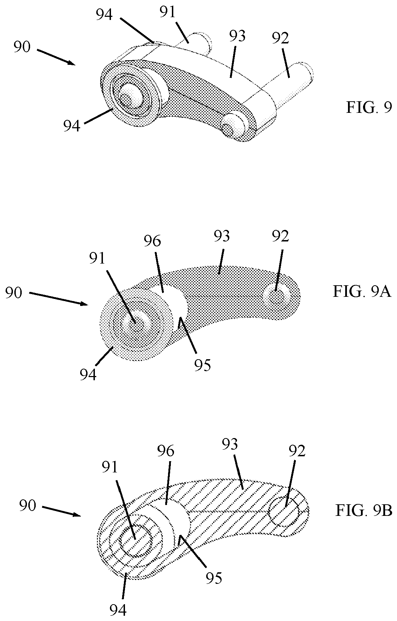

Reference is now made to Figs. 9, 9A and 9B, which illustrate a spinal implant 90 (not claimed). Here again, spinal implant 90 includes first and second cantilevered arms 91 and 92 that can be coupled to pedicle screws (the arms are structured as spinal rods to be received in the pedicle screw tulip head, for example). In contrast to the examples of Figs. 7 and 8 in which the arms are collinear with each other, in this example the arms 91 and 92 are not collinear with each other, but instead are parallel to each other, for example. Second cantilevered arm 92 is coupled to a body 93, such as being fixed to body 93 with a fastener. First cantilever arm 91 is coupled to body 93 so that it can articulate relative to body 93. For example, arm 91 may be shaped as a rod with enlarged end faces 94. Arm 91 (it is the contact member) is arranged to contact and move over a contact portion 95 of body 93, which is part of or an extension of second cantilevered arm 92. The contact portion 95 is the inner perimeter of an oval or elliptical aperture 96 formed in body 93.

## Claims

1. A spinal implant (10) comprising:
first and second pedicle screws (12, 14), each of which comprises a threaded shank (16) coupled to a head (18); and
first and second cantilevered arms (20, 22) coupled to said first and second pedicle screws (12, 14), respectively, wherein said first cantilevered arm (20) comprises a contact member (24) arranged to contact and move over a contact portion (26) of said second cantilevered arm (22), wherein said contact member (24) comprises a round roller element which rolls about pivots (38) in said first cantilevered arm (20).

2. The spinal implant (10) according to claim 1, wherein an outer contour of said head (18) is convex, and each of said first and second cantilevered arms (20, 22) comprises a concave inner portion (28), and said first and second cantilevered arms (20, 22) are secured to said first and second pedicle screws (12, 14), respectively, with a fastener (30) that presses said concave inner portion (28) against said outer contour of said head (18).

3. The spinal implant (10) according to claim 2, wherein a fastener-interface portion (34) of each of said first and second cantilevered arms (20, 22) is convex, and said fastener (30) comprises a concave inner portion (36) configured to press against said fastener-interface portion (34).

4. The spinal implant (10) according to claim 1, wherein said first and second cantilevered arms (20, 22) are parallel to each other.

5. The spinal implant (10) according to claim 1, wherein said contact member (24) is convex and said contact portion (26) is concave.

6. The spinal implant (10) according to claim 1, wherein said contact member (24) comprises a cylindrical roller bearing element which rolls about said pivots (38) and which fits in a recess (40) formed in said first cantilevered arm (20).

7. The spinal implant (10) according to claim 1, wherein said second cantilevered arm (22) comprises at least one side wall (42) that straddles said contact member (24).

8. The spinal implant (10) according to claim 1, wherein said first and second cantilevered arms (20, 22) are each pivoted about a pivot (60) with respect to a base member (62) that fits over said head (18).

9. The spinal implant (10) according to claim 1, wherein said contact member (24) has a different hardness than said contact portion (26).

## Patentansprüche

1. Wirbelsäulenimplantat (10), umfassend:
eine erste und eine zweite Pedikelschraube (12, 14), die jeweils einen Gewindeschaft (16) umfassen, der an einen Kopf (18) gekoppelt ist, und
einen ersten und einen zweiten Kragarm (20, 22), die jeweils an die erste und die zweite Pedikelschraube (12, 14) gekoppelt sind, wobei der erste Kragarm (20) ein Kontaktglied (24) umfasst, das dazu angeordnet ist, einen Kontaktabschnitt (26) des zweiten Kragarms (22) zu kontaktieren und sich über diesen zu bewegen, wobei das Kontaktglied (24) ein rundes Rollenelement umfasst, das um Drehzapfen (38) in dem ersten Kragarm (20) rollt.

2. Wirbelsäulenimplantat (10) nach Anspruch 1, wobei eine äußere Kontur des Kopfs (18) konvex ist und der erste und der zweite Kragarm (20, 22) jeweils einen konkaven inneren Abschnitt (28) umfassen und der erste und der zweite Kragarm (20, 22) jeweils mit einem Befestigungselement (30), das den konkaven inneren Abschnitt (28) gegen die äußere Kontur des Kopfs (18) drückt, an der ersten und der zweiten Pedikelschraube (12, 14) befestigt sind.

3. Wirbelsäulenimplantat (10) nach Anspruch 2, wobei ein Befestigungselement-Schnittstellenabschnitt (34) jeweils des ersten und des zweiten Kragarms (20, 22) konvex ist und das Befestigungselement (30) einen konkaven inneren Abschnitt (36) umfasst, der dazu ausgestaltet ist, gegen den Befestigungselement-Schnittstellenabschnitt (34) zu drücken.

4. Wirbelsäulenimplantat (10) nach Anspruch 1, wobei der erste und der zweite Kragarm (20, 22) parallel zueinander verlaufen.

5. Wirbelsäulenimplantat (10) nach Anspruch 1, wobei das Kontaktglied (24) konvex ist und der Kontaktabschnitt (26) konkav ist.

6. Wirbelsäulenimplantat (10) nach Anspruch 1, wobei das Kontaktglied (24) ein zylindrisches Rollenlagerelement umfasst, das um die Drehzapfen (38) rollt und das in eine in dem ersten Kragarm (20) ausgebildete Aussparung (40) passt.

7. Wirbelsäulenimplantat (10) nach Anspruch 1, wobei der zweite Kragarm (22) mindestens eine Seitenwand (42) umfasst, die das Kontaktglied (24) überspannt.

8. Wirbelsäulenimplantat (10) nach Anspruch 1, wobei der erste und der zweite Kragarm (20, 22) jeweils um einen Drehzapfen (60) bezüglich eines Basisglieds (62), das über den Kopf (18) passt, geschwenkt werden.

9. Wirbelsäulenimplantat (10) nach Anspruch 1, wobei das Kontaktglied (24) eine von dem Kontaktabschnitt (26) verschiedene Härte hat.

## Revendications

1. Implant rachidien (10) comprenant :
des première et deuxième vis pédiculaires (12, 14), dont chacune comprend une tige filetée (16) accouplée à une tête (18) ; et
des premier et deuxième bras en porte-à-faux (20, 22) accouplés auxdites première et deuxième vis pédiculaires (12, 14), respectivement, ledit premier bras en porte-à-faux (20) comprenant un élément de contact (24) agencé pour entrer en contact avec une partie de contact (26) dudit deuxième bras en porte-à-faux (22) et se déplacer sur celle-ci, ledit élément de contact (24) comprenant un élément formant rouleau rond qui roule autour de pivots (38) dans ledit premier bras en porte-à-faux (20).

2. Implant rachidien (10) selon la revendication 1, un contour extérieur de ladite tête (18) étant convexe, et chacun desdits premier et deuxième bras en porte-à-faux (20, 22) comprenant une partie interne concave (28), et lesdits premier et deuxième bras en porte-à-faux (20, 22) étant fixés auxdites première et deuxième vis pédiculaires (12, 14), respectivement, avec une attache (30) qui presse ladite partie interne concave (28) contre ledit contour extérieur de ladite tête (18).

3. Implant rachidien (10) selon la revendication 2, une partie d'interface avec l'attache (34) de chacun desdits premier et deuxième bras en porte-à-faux (20, 22) étant convexe, et ladite attache (30) comprenant une partie interne concave (36) conçue pour se presser contre ladite partie d'interface avec l'attache (34).

4. Implant rachidien (10) selon la revendication 1, lesdits premier et deuxième bras en porte-à-faux (20, 22) étant parallèles l'un à l'autre.

5. Implant rachidien (10) selon la revendication 1, ledit élément de contact (24) étant convexe et ladite partie de contact (26) étant concave.

6. Implant rachidien (10) selon la revendication 1, ledit élément de contact (24) comprenant un élément de de roulement à rouleau cylindrique qui roule autour desdits pivots (38) et qui s'insère dans un renfoncement (40) formé dans ledit premier bras en porte-à-faux (20).

7. Implant rachidien (10) selon la revendication 1, ledit deuxième bras en porte-à-faux (22) comprenant au moins une paroi latérale (42) qui chevauche ledit élément de contact (24).

8. Implant rachidien (10) selon la revendication 1, lesdits premier et deuxième bras en porte-à-faux (20, 22) étant chacun disposés de manière pivotante autour d'un pivot (60) par rapport à un élément formant base (62) qui s'adapte sur ladite tête (18).

9. Implant rachidien (10) selon la revendication 1, ledit élément de contact (24) ayant une dureté différente de celle de ladite partie de contact (26).
